# EUROPEAN PATENT APPLICATION

(11) **EP 4 063 479 A1**
(43) Date of publication of application: **28.09.2022**
(21) Application number: 22156489.1
(22) Date of filing: 14.02.2022
(51) Int. Cl.: C12M 1/107, C02F 3/28, C12M 1/00, A01C 3/02, C02F 11/04, C02F 103/20

(54) **DIGESTER STRUCTURE FOR A BIOGAS PRODUCTION PLANTS**

(30) Priority: 12.03.2021 IT 202100005840
(71) Applicant: Alvus S.r.l., 39100 Bolzano BZ (IT)
(72) Inventor: Erckert, Christof, 39100 Bolzano BZ (IT)
(74) Representative: Perani & Partners S.p.A.

(57) **Abstract**

A digester structure (1) for a biogas production plant (100) comprising: a plurality of basins (10) configured to receive an amount of organic matter to generate biogas following bacterial fermentation in anaerobiosis; a base (20) associated to a support soil (2); a perimeter wall (30) extended between a lower portion (31) associated to the base (20) and an opposite upper portion (32) spaced apart from the lower portion (31) along a height direction (X-X) perpendicular to the base (20), said perimeter wall (30) with the base (20) defining a cavity (40); an inner wall (50) extended between a lower portion (51) associated to the base (20) and an opposite upper portion (52) spaced apart from the lower portion (51) along a height direction (X-X) and configured to subdivide said cavity (40) into the plurality of basins (10).

## Description

### Technical Field

The present invention relates to a digester structure for biogas production plants. Preferably, the structure concerns basins for digesters and/or storage tanks for organic material. It must be noted that the structure may be used in the industrial field for the treatment of urban organic waste, livestock manure and/or agro-industrial byproducts and/or agro-industrial sludge/residues.

### State of the Art

Generally, biogas production plants consist of one or more anaerobic digesters respectively defining a hermetically sealed and insulated tank adapted to receive the organic substance. Each tank comprises its own structure provided with a basin to receive the organic substance and a relative cover, as well as means adapted to act within the basin. According to some known embodiments, biogas production plants provide additional tanks for storing the organic matter within which it is prepared for the digester (the organic matter is kept at a certain temperature or mixed to ensure a certain uniformity) and/or stored at the end of the fermentation process to ensure its complete degradation. It must be noted that the number of digesters and of additional tanks is a function of the electrical and/or thermal power of the respective plant.

The tanks and their structures, as known, are made of steel, reinforced concrete or reinforced earth and are spread out separated from each other on a land soil, of the biogas production plant.

### Problems of the Prior Art

It must be noted that biogas production plants have several problems.

Firstly, tanks that are separated from each other and spread out over an area of land increase the overall size of the biogas production plant and thus reduce the surrounding land area. It must also be noted that the distribution of tanks over the land area also has a negative impact on the local landscape due to the relative structures.

A further disadvantage results from the overall cost of the plant, which is related to the use of large amounts of materials for the construction of the various structures as well as materials required for the construction of the connection means between the various basins placed at a certain distance from each other.

In addition, tanks that are separated from each other increase basin thermal losses, leading to an increase in costs for maintaining the digesters at the optimum temperature for the anaerobic digestion process.

### Object of the Invention

The object of the present invention is to make a digester structure for biogas production plants capable of overcoming the above-mentioned drawbacks of the prior art.

In particular, it is an object of the present invention to provide a structure capable of reducing the area of land required to construct it as well as the costs to build it.

It is a further object of the present invention to provide a structure capable of facilitating access to the basins during routine and/or maintenance operations while maintaining a high degree of safety at work.

### Advantages of the invention

Advantageously, the structure of the present invention makes it possible to reduce the environmental impact on the landscape.

Advantageously, the structure of the present invention makes it possible to construct digesters and storage tanks cheaply and to reduce the overall cost of the plant.

Advantageously, the structure of the present invention makes it possible to construct digesters and storage tanks easily while minimising the costs of installation and disposal of the structure itself.

### BRIEF DESCRIPTION OF THE DRAWINGS

Further features and advantages of the present invention will become clearer from the indicative, and thus non-limiting, description of a preferred, though not exclusive, implementation of a digester structure and biogas production plant as shown in the accompanying drawings:
- figure 1 shows a first perspective view of a digester structure according to an embodiment of the present invention;
- figure 2 shows a second perspective view of a digester structure according to the embodiment in Figure 1;
- figure 3 shows a third perspective view of a digester structure according to the embodiment in Figure 1;
- figure 4 shows a schematic top view of the digester structure according to the embodiment in Figure 1 with some parts removed to better show other parts of a biogas production plant;

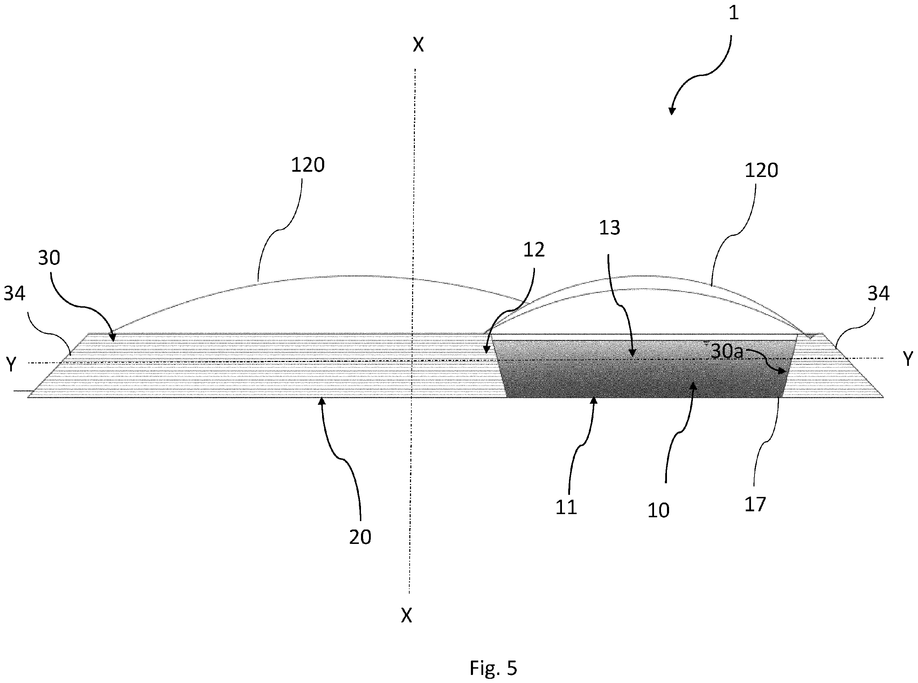

- figure 5 shows a first section view of a basin of the digester structure according to the embodiment in Figure 1 with some parts removed to better show others;

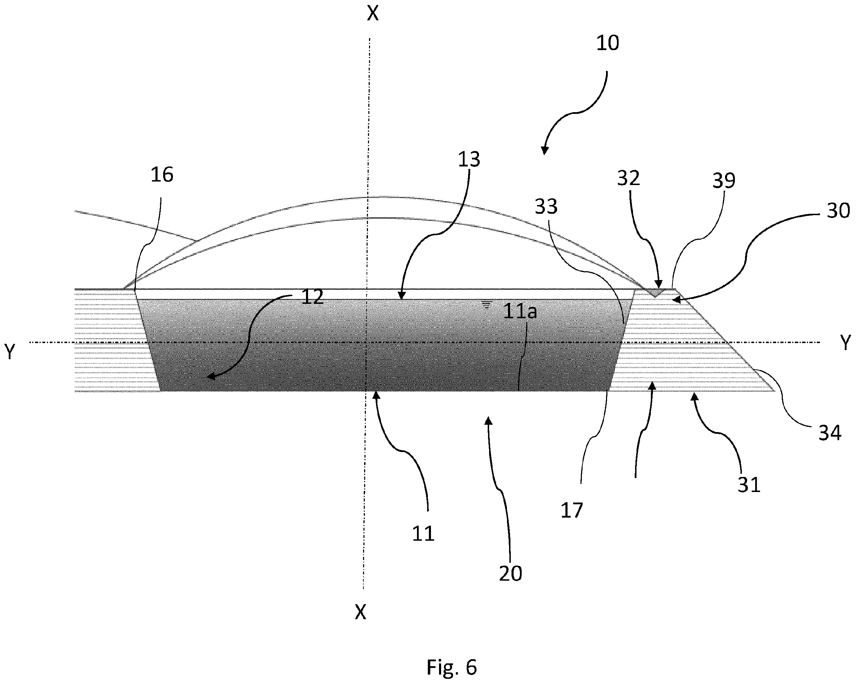

- figure 6 shows a second section view of a basin of the digester structure according to the embodiment in Figure 1 with some parts removed to better show others;

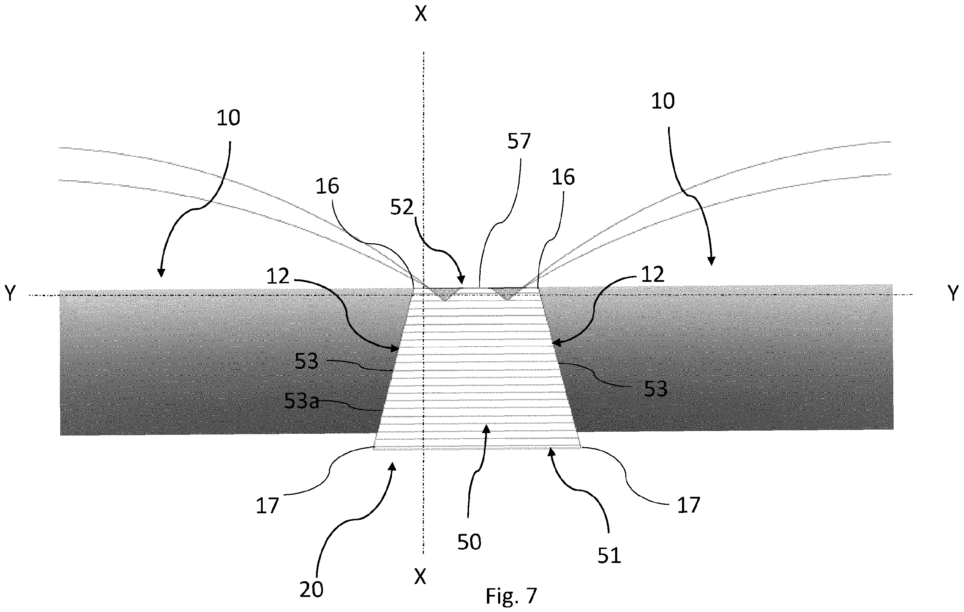

- figure 7 shows a third section view of a basin of the digester structure according to the design in Figure 1 with some parts removed to better show others.

### DETAILED DESCRIPTION

Even when not explicitly highlighted, the individual features described with reference to the specific embodiments must be considered as accessories and/or exchangeable with other features, described with reference to other embodiments.

The present invention relates to a digester structure globally referred to as 1 in the figures. This structure 1 is used in biogas production plants 100. It must be noted that for the purposes of the present invention, biogas is obtained by the bacterial fermentation in anaerobiosis (absence of oxygen) of organic residues from: decomposing organic waste, zootechnical slurry or sewage sludge or agro-industrial waste or other organic waste. It is worth noting that the plant 100 also allows bio-fertilisers (solid and liquid) to be obtained in addition to biogas. Fermentation and subsequent production of biogas and/or bio-fertilisers occurs in one or more digesters 140 and storage tanks 150.

In fact, the plants also include one or more storage tanks 150 configured to receive the processed or raw organic matter exiting the digesters before it is introduced into the digesters 140.

The structure 1 comprises a plurality of basins 10 configured to receive an amount of organic matter to generate biogas following an anaerobic bacterial fermentation. It must be noted that such basins 10 can be used both to make tanks 130 for a digester 140, as shown hereinafter, and for storage tanks 150. Each basin in the present structure can thereby fit the plant requirements.

According to a preferred embodiment, each basin 10 comprises a basin base 11 and a side wall 12 rising from said basin base 11, preferably transversely to the basin base 11 itself. The side wall 12 with the basin base 11 defines a cavity 13 configured to contain at least partially the amount of organic matter. It is worth noting that the cavity may be partially occupied by gaseous substances depending on the filling of the basin with organic material.

The structure 1 comprises a base 20 associated to a support soil 2. Specifically, the base 20 is substantially parallel to the support soil 2 and is configured to support at least partially the structure 1. Preferably, the base 20 is made of earth following appropriate levelling and/or concrete. More preferably, the base 20 represents the foundations of the structure.

The structure 1 comprises a perimeter wall 30 extended between a lower portion 31 associated to the base 20 and an opposite upper portion 32 spaced apart from the lower portion 31 along a height direction X-X perpendicular to the base 20. Specifically, the perimeter wall 30 with the base 20 defines a cavity 40. The latter is configured to be subdivided into the plurality of basins 10. The basins 10 are thereby grouped together within a single structure delimited by the perimeter wall 30 and the base 20.

The structure 1 comprises an inner wall 50 extended between a lower portion 51 associated to the base 20 and an opposite upper portion 52 spaced apart from the lower portion 51 along the heigh direction X-X. The inner wall 50 is configured to subdivide said cavity 40 into the plurality of basins 10.

Advantageously, the structure 1 thus created makes it possible to reduce the space required for the construction of basins 10. Specifically, such basins 10 are separated from each other by the inner wall 50 thus limiting the relative distance between them.

Preferably the inner wall 50 and the perimeter wall 30 subdivide the base 20 to define the basin bases 11 of the respective basins 10. Specifically, the basin bases 11 thus defined by the inner wall 50 and the perimeter wall 30 have an extension surface 11a on which the organic matter contained within the respective basin 10 can rest. This extension area 11a is preferably horizontal and parallel to the support soil 2.

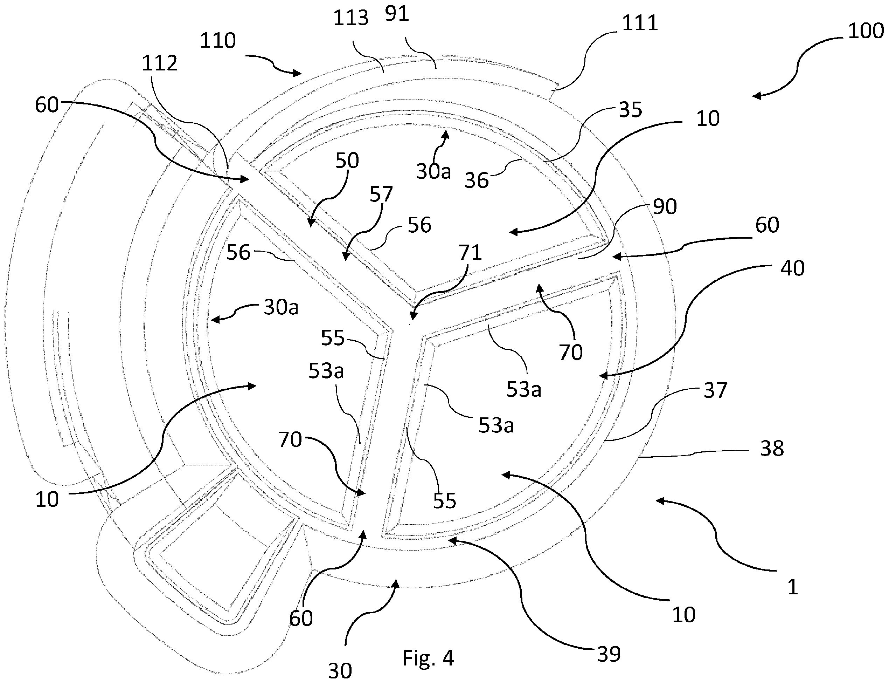

According to preferred embodiments, each basin base 11 may have different profiles such as a circular, rectangular, square, polygonal or circular-sector profile (Figure 4).

Preferably, each basin base 11 has an extension area 11a between 2000 and 2500 m².

According to a preferred embodiment, the perimeter wall 30 and the inner wall 50 define the side walls of each basin 10. Specifically, the inner wall 50 subdivides the perimeter wall 30 into perimeter sections 30a configured to define the side walls 12 of the basins 10 together with the inner wall 50.

Advantageously, the structure 1 thus made makes it possible to reduce the material required for the construction of basins 10 by exploiting the inner wall 50 to define at least partially the side wall of two adjacent basins 10.

Preferably, the perimeter wall 30 has an inner perimeter surface 33 defining at least partially the side wall 12 of each basin 10 and an opposite outer perimeter wall 34. It is worth noting that the perimeter wall 30 extends between the inner perimeter surface 33 and the outer perimeter surface 34 along a thickness direction Y-Y perpendicular to the height direction X-X for a thickness between 5 and 20 m,

In accordance with a preferred embodiment, the inner perimeter surface 33 and the outer perimeter surface 34 rise from the base along transverse directions approaching each other from the lower portion 31 to the upper portion 32 along the height direction X-X. In other words, the inner perimeter surface 33 and the outer perimeter surface 34 are tilted with respect to the height direction X-X as well as with respect to the base 20. Specifically, the perimeter wall 30 tapers from the lower portion 31 to the upper portion 32. Preferably, the inner perimeter surface 33 defines with the base 20 a first obtuse angle between 90° and 135°, while the outer perimeter surface 34 defines with the base 20 and/or the support soil 2 a second obtuse angle between 100° and 150°. According to a preferred embodiment, the obtuse angle between the perimeter surfaces 33, 34 and the base 20 is equal.

In terms of height extension, the perimeter wall 30 has a height measured between the base 20 and the relative upper portion 32 along the height direction between 8 and 12 m.

As regards the inner wall 50, this is connected to the inner perimeter surface 33 at joint sections 60, preferably extending along the inner perimeter surface 33 from the lower portion 31 to the upper portion 32. In other words, the inner wall 50 extends from the inner perimeter surface 33 towards a portion of the inner perimeter surface 33 facing thereto within the cavity 40. Specifically, the inner wall 50 has inner surfaces 53 defining at least partially the side walls 12 of each basin 10. In detail, the inner wall 30 has inner surfaces 53 spaced apart along the thickness direction Y-Y defining adjacent basins 10. In detail, the side wall 50 separates two adjacent basins 10. It is worth noting that, in terms of thickness, the inner wall 50 extends between two opposite inner surfaces 53 spaced apart along the thickness direction Y-Y for a thickness between 8 and 15 m.

Regarding the height, the inner wall 50 has a height measured between the base 20 and the relative upper portion 52 between 8 and 12 m.

According to a preferred embodiment, the inner surfaces 53 rise from the base along transverse directions approaching each other from the lower portion 51 to the upper portion 52 along the height direction X-X. In other words, the inner surfaces 53 are tilted with respect to the height direction X-X as well as with respect to the base 20. Specifically, the inner wall 50 tapers from the lower portion 51 to the upper portion 52. Preferably, each inner surface 53 defines with the base 20 an obtuse angle between 90° and 135°.

According to the aforementioned embodiments wherein the perimeter wall 30 and the inner wall 50 have tilted surfaces, the side wall 12 of each basin 10 is tilted along the same transverse directions as the surfaces defining it.

Advantageously, the tilted surfaces 33, 34, 53 allow for an easier design and construction of the structure 1.

It is worth noting that in alternative embodiments the perimeter surfaces 33, 34 and the inner surfaces 53 may be perpendicular to the base or may be partially tilted and partially perpendicular to the base. For example, the outer perimeter surface 34 may be perpendicular to the base 20 and the inner perimeter surface 33 and the inner surfaces 53 may be tilted with respect to the base 20.

According to a preferred embodiment, the inner wall 50 subdivides the perimeter wall 30 into perimeter sections 30a extended between consecutive joint sections 60 so as to define at least partially the side wall of a basin 10. It must be noted that each perimeter section 30a is a portion of the inner perimeter surface 33, which defines, together with the inner wall 50 defining the relevant perimeter section 30a, the side wall 12 of a basin 10.

According to a preferred embodiment, the inner wall 50 has one or more inner wall segments 70 connected to each other in one or more connection areas 71 preferably arranged within the cavity 40 and/or coinciding with the joint sections 60. Specifically, each inner wall segment 70 extends from a related junction section 60 to the connection area 71 separating and defining their adjacent basins 10. The inner wall segments 70 thereby subdivide the perimeter wall 30 into the perimeter sections 30a each associated to a relative basin 10.

It is worth noting that each segment of inner wall 70 defines with other segments of inner wall 70 and/or with the perimeter wall 30 the side wall 12 of each basin 10.

According to the embodiment shown in the figures, the inner wall 30 has a first segment 72, a second segment 73 and a third segment 74 which, connected at the connection area 71 within the cavity 40, divide it into three basins 10. Thus, segments 72, 73, 74 subdivide the perimeter wall into three perimeter sections 30a each extending between the junction sections 60 of two consecutive segments. The perimeter wall 30 together with the inner wall 50 thereby define the side walls of each basin 10.

Preferably, the inner surfaces 53 of the inner wall 50 have inner wall portions 53a respectively facing adjacent basins 10 and defining at least partially the side wall 12 of the respective basins 10. It is worth noting that each side wall section 70 is associated to a portion of inner wall 53a for each basin 10 that the relevant section 70 separates.

Specifically, the side wall 12 of each basin 10 is defined by the perimeter section 30a defined by the inner wall 50, preferably defined by one or more sections of inner wall 70, and the entire surface 53 facing the respective perimeter section 30a. In detail, the side wall 12 of each basin 10 is defined by the perimeter section 30a and by one or more inner wall portions 53a facing the respective perimeter section 30a.

According to a preferred embodiment, the inner perimeter surface 33 defines at the upper portion 32 of the perimeter wall 30 an inner upper perimeter edge 35 and at the lower portion 32 an inner lower perimeter edge 36. Preferably the upper inner perimeter edge 35 and the lower inner perimeter edge 36 have a circular profile respectively.

Furthermore, the outer perimeter surface 34 also defines at the upper portion 31 of the perimeter wall 30 an outer upper perimeter edge 37 and at the lower portion 32 an outer lower perimeter edge 38 which may have different profiles such as circular, rectangular or square.

It is worth noting that in the embodiment shown in the figures, the defined cavity 40 has an inverted truncated cone shape, i.e. with a larger side facing the base 20 and a smaller side associated to the base 20, while the perimeter wall 30 has a truncated cone shape, i.e. with a larger side associated to the base 20 and a smaller side facing the base 20. More specifically, the structure 1 shown in the figures has an average inner radius between 50 and 55 m (measured with respect to the inner perimeter surface 34) and an average outer radius between 65 and 75 m (measured with respect to the outer perimeter surface 35).

According to alternative embodiments, the perimeter edges 35, 36, 37, 38 can take on different profiles such as square, rectangular or polygonal giving the cavity 40 and the perimeter wall 30 different shapes.

Preferably, the inner surface 53 defines at the upper portion 52 an inner upper edge 55 and at the lower portion 51 an inner lower edge 56. It is worth noting that the inner upper edge 55 and lower inner edge 56 are connected to the inner upper perimeter edge 35 and inner lower perimeter edge 36 respectively at the connection sections 60. Specifically, the upper edges 35, 55 and the lower edges 36, 56 define an upper edge 16 and a lower edge 17 of each basin 10 into which they subdivide the cavity 50.

Specifically, each upper edge 16 of the basins 10 delimits a respective opening of the basin cavity 13 spaced apart from the respective basin base along the height direction X-X. In contrast, the lower edge 17 delimits the profile of the basin base 11, as previously shown.

Preferably, the upper edge 16 and the lower edge 17 have the same profile. According to alternative embodiments, the upper edge 16 and the lower edge 17 may have a different profile.

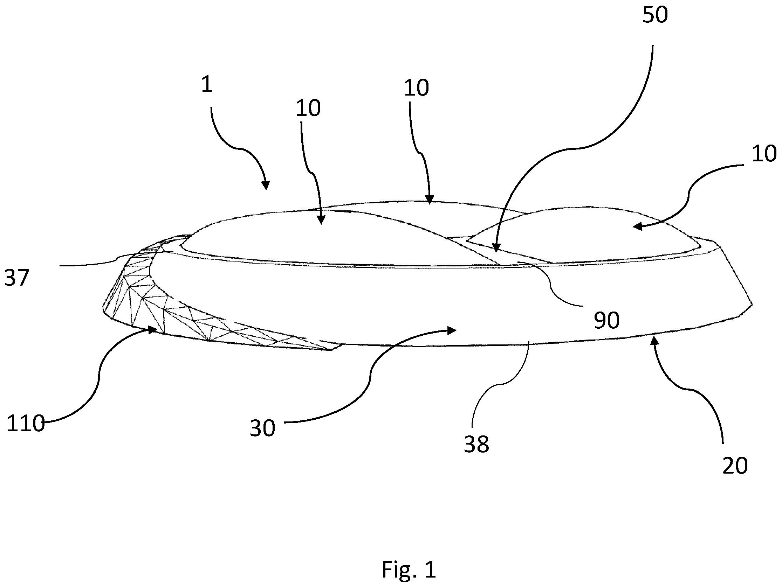

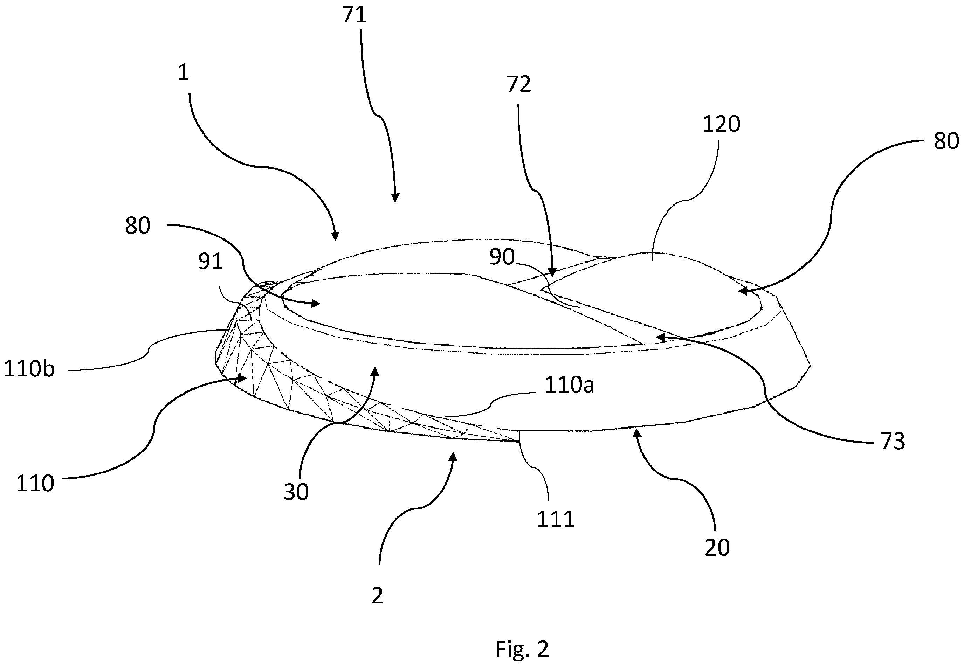

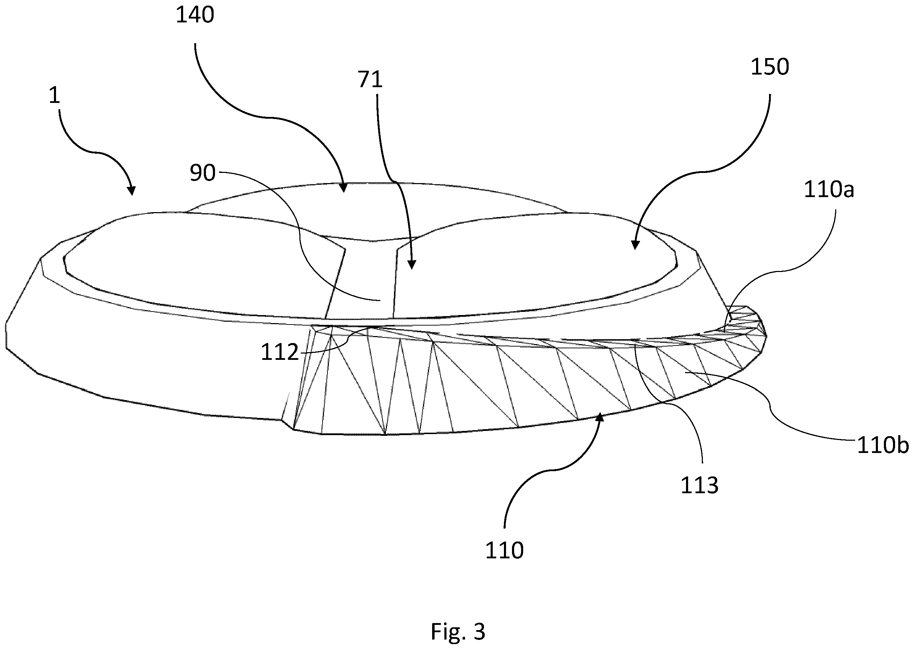

According to a preferred embodiment shown in the figures, the inner wall 50 is configured to subdivide the inner upper perimeter edge 35 and the inner lower perimeter edge 36 into two or more circular sectors 80 (Figures 1-3). Specifically, the inner upper edge 55 and the inner lower edge 56 define basins having upper edges 16 and lower edges 17 in the shape of a circular sector 80. In other words, the inner wall 50 and the perimeter wall 30 define basins 10 having a section transversal to the height direction having a circular sector shape or also called "sliced". Preferably, the structure 1 has three substantially equal circular sectors 80 having a first extension measured along the inner wall sections 70 between the junction section 60 and the connection area 71 between 50 and 60 m and a second extension measured along a transverse direction between the junction sections 60 defining the respective perimeter section 30a between 90 and 100 m. Specifically, each circular sector cavity 80 has a volume between 20,000 and 30,000 m³.

According to alternative embodiments, not shown in the figures, the upper edge 16 and the lower edge 17 of the basins 10 have different shapes such as circular, rectangular or square or polygonal profiles.

According to a preferred embodiment, the perimeter wall 30 at the relative upper portion 33 comprises a perimeter support surface 39 connected to the inner perimeter surface 33 at the outer perimeter surface 34. Preferably, such perimeter support surface 39 extends between the inner upper perimeter edge 35 and the outer upper perimeter edge 37. According to the present embodiment, the inner wall 50 at the relative upper portion 52 comprises an inner support surface 57 connected to the inner surfaces 53 associated to adjacent basins 10 and connected to the perimeter support surface 39 at intersection points 60. Preferably, said inner support surface 57 extends between inner upper edges 55 associated to adjacent basins 10. It is worth noting that the inner support surface 57 is subdivided between the inner wall segments 70.

Preferably, the perimeter support surface 39 and the inner support surface 57 are joined at the joint sections 60.

More preferably, the perimeter support surface 39 and the inner support surface 57 are permanently flat and parallel to the base 20. Even more preferably, the perimeter support surface 39 and the inner support surface 57 lie substantially on a same plane parallel to the base 20.

It is worth noting that the perimeter support surface 39 has a width between the inner perimeter surface 33 and the outer perimeter surface 34, preferably between the perimeter edges 35, 37 between 3 and 5 m. By contrast, the inner support surface 57 has a width between the inner surfaces 53 associated to adjacent basins 10, preferably between the inner upper edges of adjacent basins 10, between 7 and 9 m.

According to a preferred embodiment, the perimeter support surface 39 and the inner support surface 57 define motor vehicle movement paths 90 between the basins 10. These paths allow and ease not only vehicles, but also operators to access the basins 10 for operations on the basins as well as on the digesters and/or storage tanks.

According to a preferred embodiment shown in the figures, the support structure is provided with an access ramp 110 configured to connect the upper portion 31 of the perimeter wall 30 to the base 20. Specifically, the access ramp is configured to connect the base 20 to the movement paths 90. In detail, the access ramp 110 defines an access path 91 that allows vehicles and operators to reach the movement paths 90 easily.

Preferably, the access ramp 110 at least partially surrounds the outer perimeter surface 34. Specifically, the access ramp 110 extends from the base 20 along a substantially helical path around the outer perimeter surface 34. In detail, the access ramp 110 has a first support wall 110a obtained on the outer perimeter surface 34 and an opposite second support wall 110b. Such support walls 110a, 110b are configured to support the weight carried on the access ramp 110. Preferably, the support walls 110a, 110b are tilted according to the tilt of the outer perimeter surface 34 to better adapt the access ramp 110 to the perimeter wall 30 and facilitate construction thereof.

It is worth noting that the access path 91 has an initial portion 111 near the base 20 and substantially at the level of the support soil 2, a final portion 112 near the upper portion 31 of the side wall 30 and an intermediate portion 113 extending between the initial portion 111 and the final portion 112. Such intermediate portion 113 is tilted with respect to the base 20 and has an extension around the outer perimeter surface 34 depending on the height of the perimeter wall 30 and the width of the cavity 40.

More preferably, the access ramp 110 and thus the access path 91 have a width measured between the support walls 110a, 110b near the intermediate portion 113 between 5 and 6 m.

According to a preferred embodiment, one or more basins 10 are provided with a cover membrane 120 mechanically fixed to the corresponding perimeter wall 30 and inner wall 50, preferably at their respective upper edges. Specifically, each cover membrane 120 defines with the relative basin 10 a digester 140. It is worth noting that each digester 140 has a tank defined by the basin 10 and the relative cover membrane. Preferably, a relative cover membrane 120 is associated to each basin 10, thus allowing direct and easy access to the relative basin 10. Preferably, the cover membranes 120 associated to the relative basins 10 are mechanically fixed at the upper portion of the inner wall 50 and at the upper portion 32 of the perimeter wall 30. Specifically, the cover membranes 120 are mutually separated from the upper portion 52 of the inner wall 50 and the upper portion 32 of the perimeter wall 30. It is worth noting that each cover membrane 120 is therefore separated from the adjacent cover membranes 120 by the inner support surface 57 and the perimeter support surface 39. In other words, each cover membrane 120 is surrounded at the upper portions 32, 52 by the inner support surface 57 and the perimeter support surface 39. Each basin 10 is thereby individually and directly accessible for operations of a different nature, such as for example maintenance, control and/or routine operations, at least by means of the relative cover membrane 120 from the movement paths 90 associated to the inner support surface 57. In addition, thanks to the cover membranes 120 being associated to the relative basins 10, access to the basin 10, for example by the cover membrane 120 is also easily achieved via the movement paths 90 also associated to the perimeter support surface 39. Preferably, each cover membrane 120 is associated to a relative basin 10 and is configured to allow access to the relative basin 10 from the upper portion 52 of the inner wall 50 and from the upper portion 32 of the perimeter wall 30, more preferably from the relative support surfaces 57, 39.

In alternative or in combination with the above embodiment, the cover membrane 120 defines with the basin 10 a storage tank 150.

Such a membrane cover 120 is configured to collect the biogas generated by the organic matter contained within the basin. Specifically, the biogas leaving the organic matter is enclosed between the latter and the membrane cover 120. It must be noted that according to a preferred embodiment, the side wall 12 of each basin is lined with layers suitable for the storage and fermentation of organic material.

According to a preferred embodiment, the structure 1 is made of reinforced earth, reinforced concrete or a combination of both. Preferably, the perimeter wall 30 and the inner wall 50 are made of reinforced earth, reinforced concrete or a combination thereof. It is worth noting that the structure 1 made of reinforced earth allows to make a perimeter wall 30 and an inner wall 50 of the structure 1, reducing costs and simplifying construction.

According to a preferred embodiment, the structure comprises one or more circuits connecting the basins 10 and outer basins for managing the organic material and biogas produced.

Advantageously, the structure 1 of the present invention may be used in biogas production plants capable of producing an hourly amount of biogas between 250 and 2500 Sm³/h.

The present invention also relates to a biogas production plant 100 provided with the digester structure 1. The plant 100 comprises the digester structure 1 defining one or more storage tanks 150, at least one digester 140 and one or more circuits configured to move the organic material between the storage tanks 150 and the digesters 140 and to move the biogas produced by the digesters 140 and the storage tanks 150 to users.

According to a preferred embodiment, the structure 1 comprises at least one basin 10 configured to define a storage tank 150 and at least one basin 10 configured to define a digester 140 placed in fluid communication with each other by means of a relative circuit.

The plant 100 is provided with an additional circuit for recovering the biogas produced associated to the digester 140 and the storage tank 150 so as to make the biogas available to one or more users configured to generate electrical and/or thermal energy from the latter.

## Claims

1. Digester structure (1) for a biogas production plant (100) comprising:
- a plurality of basins (10) configured to receive an amount of organic matter to generate biogas following an anaerobic bacterial fermentation;
**characterised in that** it comprises
- a base (20) associated to a support soil (2);
- a perimeter wall (30) extended between a lower portion (31) associated to the base (20) and an opposite upper portion (32) spaced apart from the lower portion (31) along a heigh direction (X-X) perpendicular to the base (20), said perimeter wall (30) with the base (20) defining a cavity (40);
- an inner wall (50) extended between a lower portion (51) associated to the base (20) and an opposite upper portion (52) spaced apart from the lower portion (51) along the heigh direction (X-X) and configured to subdivide said cavity (40) into the plurality of basins (10).

2. Digester structure (1) according to claim 1, wherein:
- each basin (10) comprises a basin base (11) and a side wall (12) rising from said basin base (11) and defining with the basin base (11) a basin cavity (13) configured to contain at least partially the amount of organic matter,
- the inner wall (50) and the perimeter wall (30) subdivide the base (20) to define the basin bases (11) of the respective basins (10)

3. Digester structure (1) according to claim 1 or 2 wherein:
- the perimeter wall (30) has an inner perimeter surface (33) defining at least partially the side wall (12) of each basin (10) and an opposite outer perimeter wall (35);
- the inner part (50) is connected to the inner perimeter surface (33) at joint sections (60) and has an inner surface (53) defining at least partially the side walls (12) of each basin (10).

4. Digester structure (1) according to claim 3, wherein
- the inner wall (50) has one or more inner wall segments (70) connected between each other in one or more connection areas (71) arranged inside the cavity (50), each inner wall segment (70) defining with other inner wall segments (70) and/or with the perimeter wall (30) the side wall (12) of each basin (10), each inner wall segment (70) separating adjacent basins (10) from each other.

5. Digester structure (1) according to claim 2 or 3 wherein:
- the inner perimeter surface (33) defines at the upper portion (31) of the perimeter wall (30) an inner upper perimeter edge (35) and at the lower portion (32) an inner lower perimeter edge (36), the inner upper perimeter edge (35) and the inner lower perimeter edge (36) having respectively a circular profile,
- the inner wall (50) is configured to subdivide the inner upper perimeter edge (35) and the inner lower perimeter edge (36) in two or more circular sectors (80).

6. Digester structure (1) according to any one of claims 2 to 5, wherein
- the perimeter wall (30) at the relative upper portion (31) comprises a perimeter support surface (39) connected to the perimeter surface (33) internal to the outer perimeter surface (34);
- the inner wall (50) at the relative upper portion (51) comprises an inner support surface (57) connected to the inner surfaces (53) associated to adjacent basins (10) and connected to the perimeter support surface (39) at intersection points (60);
- the perimeter support surface (39) and the inner support surface (57) define motor vehicle movement paths (90).

7. Digester structure (1) according to claim 6, wherein:
- the perimeter support surface (39) has a width between the inner perimeter surface (33) and the outer perimeter surface (34) between 3 and 5 m;
- the inner support surface (57) has a width between the inner surfaces (53) associated to adjacent basins (10) between 7 and 9 m

8. Support structure (1) according to any one of claims 1 to 7, wherein the support structure is provided with an access ramp (110) configured to connect the upper portion (31) of the perimeter wall (30) to the base (20) at the support soil (2), said access ramp (110) surrounding at least partially the outer perimeter surface (34) of the perimeter wall (30).

9. Digester structure (1) according to any one of claims 1 to 8, wherein one or more basins (10) are provided with a cover membrane (120) mechanically fixed to the corresponding perimeter wall (30) and the inner wall (50), each cover membrane (120) defining with the relative basin (10) a tank (130) for a digester (140).

10. Digester structure (1) according to claim 9, wherein the cover membranes (120) associated to the relative basins (10) are mechanically fixed at the upper portion of the inner wall (50) and at the upper portion (32) of the perimeter wall (30), the cover membranes (120) being mutually separated from the upper portion (52) of the inner wall (50) and the upper portion (32) of the perimeter wall (30).

11. Digester structure (1) according to claim 9 or 10, wherein each cover membrane (120) associated to a relative basin (10) is configured to allow access to the relative basin (10) from the upper portion (52) of the inner wall (50) and from the upper portion (32) of the perimeter wall (30).

12. Digester structure (1) according to any one of the preceding claims wherein the perimeter wall (30) and the inner wall (50) are made of reinforced earth, reinforced concrete or a combination thereof.
